# EUROPEAN PATENT SPECIFICATION

(11) **EP 0 806 215 B1**
(45) Date of publication and mention of the grant of the patent: **30.06.2004**
(21) Application number: 97301085.3
(22) Date of filing: 20.02.1997
(51) Int. Cl.: A61M 16/01

(54) **Apparatus for recovering an anaesthetic agent**
Vorrichtung zur Rückgewinnung von Narkosemittel
Dispositif pour la récupération d'agent anesthésique

(30) Priority: 17.04.1996 GB 9607968
(43) Date of publication of application: 12.11.1997
(73) Proprietor: The BOC Group plc, Windlesham Surrey GU20 6HJ (GB)
(72) Inventor: Garrett, Michael Ernest, Surrey, GU22 7XR (GB); Hughes, Ronald, Stockport, SK7 3BU (GB)
(74) Representative: MacLean, Martin David

(56) References cited:
- WO-A-94/02190
- DE-A- 4 340 764
- DE-C- 3 513 628
- US-A- 3 592 191

## Description

The present invention relates to an apparatus for recovering an anaesthetic agent and relates particularly, but not exclusively, to such an apparatus for recovering anaesthetic agent exhaled by a patient. The invention also relates to a method of recovering anaesthetic agent exhaled by a patient.

Many anaesthetic agents used to anaesthetise patients during invasive surgery are exhaled by the patients into the atmosphere immediately surrounding him and can therefore be inhaled by the operating staff immediately adjacent thereto. Such quantities of anaesthetic agent whilst being comparatively small, nevertheless present an uncertain long term effect on operating personnel. Consequently, there exists a requirement for a method of separating the anaesthetic gas remaining in a patients exhalation and preventing it from being inhaled by the operating personnel.

One method of separating the anaesthetic gas is described in US-A-3 592 191 which includes condensation of the vapours in the anaesthetic agent to a liquid phase.

In accordance with the present invention there is provided an apparatus for recovering an anaesthetic agent exhaled from a patient, according to claim 1.

A method for recovering an anaesthetic agent exhaled by a patient is disclosed in claim 10.

Preferably, the region comprises an anaesthetic supply means for supplying anaesthetic to the patient, thereby to provide for recycling of said separated anaesthetic agent.

Advantageously, the apparatus further includes disposal means operably connected to the first outlet of the separation means or directing pressurised gas depleted the anaesthetic agent for release to atmosphere at a point remote from the atmosphere surrounding the patient.

Preferably, the apparatus includes pressure sensor means for monitoring the pressure within the buffer and for causing operation of the compressor only upon detection of a pressure above about atmospheric pressure.

Conveniently, the apparatus further includes control means for receiving a signal from said pressure monitor and for initiating control over the operation of the compressor as and when necessary.
Preferably, the compressor is operable to compress the exhaled gas to a pressure equal to or greater than 1 atmosphere gauge.

Advantageously, the apparatus includes a gas analyser operably connected to monitor the purity of separated anaesthetic agent and being operably connected to an anaesthetic supply apparatus such as to enable recycled anaesthetic agent to be re mixed with make-up oxygen and fresh anaesthetic agent for supply to the patient.

Conveniently, the buffer vessel comprises a collapsible/expandable bag buffer.

In one particular arrangement, the anaesthetic agent comprises nitrous oxide.

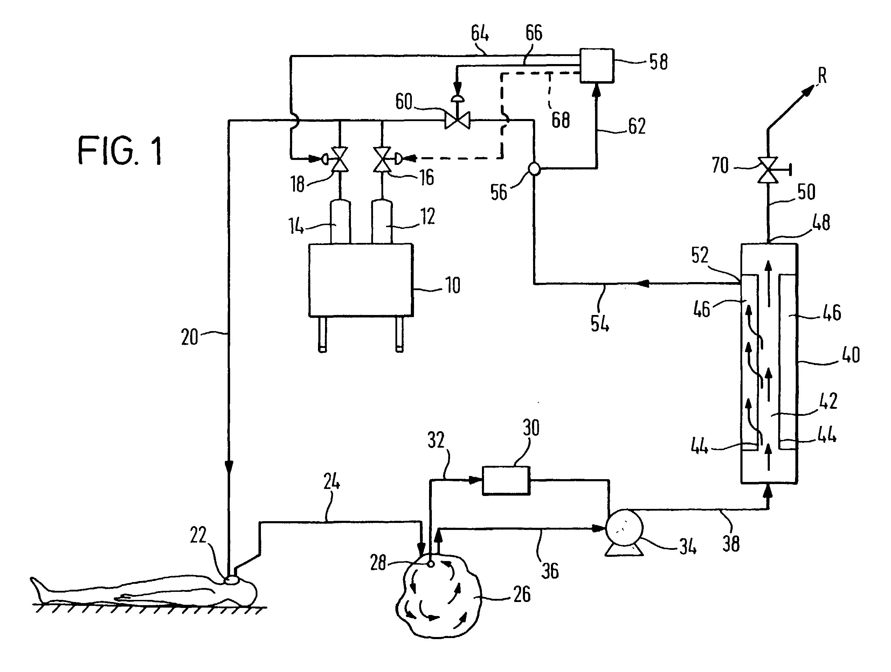

The present invention will now be more particularly described by way of example only with reference to and as illustrated in figure 1 attached hereto which is a schematic representation of the present invention.

From figure 1 it will be seen that an anaesthetic supply apparatus 10 supplies oxygen 12 and anaesthetic agent 14 to control valves 16 and 18 before these components are mixed and directed along supply line 20 to a patients breathing mask 22. During sedation, the patients exhalation is withdrawn via line 24 and directed to a buffer vessel 26 in the form of, for example, a collapsible/expandable bag buffer. A pressure sensor 28 acts to a monitor the pressure within the buffer 26 and provides a signal to a pump controller 30 via line 32. A pump 34 situated downstream of buffer 26 is operably linked to pump controller 30 which initiates control and operation of the pump only upon detection of a pressure within the buffer equal to or greater than 1 atmosphere gauge. Line 36 is provided for withdrawing exhalation from buffer 26 and directing it to pump 34. Once compressed, the exhalation is directed via line 38 to a separation apparatus shown generally at 40. The separation apparatus 40 comprises a membrane separation apparatus operably connected to receive compressed exhaled gas to its tube side 42 and being configured for allowing said anaesthetic agent to pass through the membrane 44 to the shell side 46 thereof from which it may be withdrawn in a manner to be described later herein. A first outlet 48 is provided for receiving pressurised gas depleted in anaesthetic agent and for directing it to line 50 for subsequent disposal. A second outlet 52 is provided for receiving separated anaesthetic agent and for directing it to line 54 which conducts the separated anaesthetic agent to a region other than the atmosphere surrounding the patient. Whilst it will be appreciated that this region might be any area other than that in which the operating staff are situated, it will conveniently comprise the anaesthetic supply apparatus itself in which case the recycled anaesthetic agent may be used once again. In this arrangement, it will be necessary to provide a gas analyser purity monitor 56 and control apparatus 58 which initiates control over the introduction of recycled anaesthetic via valve 60 and modifies the flow rate of new anaesthetic agent via control valve 18. If necessary, control may also be initiated over oxygen supply valve 16. For the purposes for this control, line 62 is provided for supplying a signal to controller 58 from monitor 56 and lines 64 to 68 are provided for initiating control over valves 16, 18 and 60. Once remixed with fresh anaesthetic agents and oxygen, the recycled anaesthetic is once again supplied to the patient via line 20. Whilst it will be appreciated that little if any anaesthetic agent will remain in the gas supplied to first outlet 48, and that it will therefore not present a major hazard to the operating staff, this gas stream is conveniently discharged to atmosphere in a region other than the atmosphere surrounding the patient. A control valve 70 may be provided in outlet line 50.

If necessary, pump 34 may be operated to pressurise the exhaled gas to a pressure somewhat greater than that described above thereby facilitating the provision of compressed recycled anaesthetic agent to anaesthetic supply apparatus 10. A further advantage of the present invention resides in the fact that items such as dust and bacteria will be retained within the membrane rather than past pack to the anaesthetic supply. Such a membrane may therefore be used on a number of patients without presenting a biological hazard. Alternatively, such separation apparatus are comparatively inexpensive to produce and hence a fresh unit may be provided for each and every patient.

Whilst it will be appreciated that the present invention may be employed with any one a number of anaesthetic agents, it has been found to be particularly suitable for use with nitrous oxide.

## Claims

1. Apparatus for recovering an anaesthetic agent exhaled from a patient, the apparatus comprising:
a buffer vessel (26), for receiving a patient's exhalation and for temporarily storing the same;
separation means (40), for receiving and for directing the separated anaesthetic agent to a region other than the atmosphere surrounding the patient exhaled gas and for separating the anaesthetic agent therefrom, **characterised in that** the apparatus has a compressor (34), downstream of the buffer vessel (26) and operably connected thereto for receiving and compressing exhaled gas and **in that** the separation means comprises a membrane separation apparatus operably connected to the compressor (34) to receive compressed exhaled gas to its tube side and being configured for allowing the anaesthetic agent to pass through the membrane (44) to the shell side (46) thereof, the separation means further including a first outlet (48) for receiving pressurised gas depleted in anaesthetic agent and a second outlet (52) for receiving the separated anaesthetic agent.

2. An apparatus as claimed in claim 1 in which said region comprises an 2. An apparatus as claimed in Claim 1 in which said region comprises an anaesthetic supply means for supplying anaesthetic to the patient, thereby to provide for recycling of said separated anaesthetic agent.

3. An apparatus as claimed in Claim 1 or Claim 2 including disposal means operably connected to the first outlet (48) of the separation means for directing pressurised gas depleted in anaesthetic agent for release to atmosphere at a point remote from the atmosphere surrounding the patient.

4. An apparatus as claimed in any one of Claims 1 to 3 including pressure sensor means (28) for monitoring the pressure within the buffer vessel (26) and for causing operation of the compressor (34) only upon detection of a pressure above about atmospheric pressure.

5. An apparatus as claimed in Claim 4 further including control means (30) for receiving a signal from said pressure sensor means (28) and for initiating control over the operation of the compressor as and when necessary.

6. An apparatus as claimed in any one of Claims 1 to 5 in which the compressor (34) is operable to compress the exhaled gas to a pressure equal to or greater than one atmosphere gauge.

7. An apparatus as claimed in any one of Claims 1 to 6 including a gas analyser (56) operably connected to monitor the purity of separated anaesthetic agent and being operably connected to an anaesthetic supply apparatus (10) such as to enable recycled anaesthetic agent to be re mixed with make-up oxygen and fresh anaesthetic agent for supply to the patient.

8. An apparatus as claimed in any one of Claims 1 to 7 in which the buffer vessel (26) comprises a collapsible / expandable bag buffer.

9. An apparatus as claimed in any one of Claims 1 to 8 in which the anaesthetic agent comprises nitrous oxide.

10. A method of recovering an anaesthetic agent exhaled by a patient, the method comprising receiving and temporarily storing a patient's exhalation in a buffer vessel (26); directing the exhaled gas from the buffer vessel (26) to a separation apparatus (40) thereby to separate the anaesthetic agent from the exhaled gas and directing the separated anaesthetic agent to a region other than the atmosphere surrounding the patient, **characterised in that** the exhaled gas from the buffer vessel (26) is directed to a compressor (34) compressing the exhaled gas, and from the compressor is directed to the separation apparatus (40) comprising a membrane separation apparatus.

## Patentansprüche

1. Gerät zur Rückgewinnung eines von einem Patienten ausgeatmeten anästhetischen Mittels, mit:
einem Pufferbehälter (26) zur Aufnahme der Ausatmung der Patienten und zur zeitweisen Speicherung derselben,
Trennmitteln (40) zur Aufnahme von ausgeatmetem Gas und zum Trennen des anästhetischen Mittels hiervon und zum Leiten des abgetrennten anästhetischen Mittels zu einem anderen Bereich als in die dem Patienten umgebende Atmosphäre,
**dadurch gekennzeichnet, daß** das Gerät einen Verdichter (34) aufweist, der stromab des Pufferbehälters (26) angeordnet und betriebsmäßig zur Aufnahme und Verdichtung von ausgeatmetem Gas damit verbunden ist, und daß die Trennmittel ein Membrantrenngerät umfassen, das mit dem Verdichter (34) zur Aufnahme von verdichtetem ausgeatmetem Gas in seiner Rohrseite betriebsmäßig verbunden und so konfiguriert ist, daß das anästhetische Mittel durch die Membran (44) zur Gehäuseseite (46) hindurchtreten kann, wobei die Trennmittel des weiteren einen ersten Auslaß (48) zur Aufnahme von an anästhetischem Mittel erschöpftem druckbeaufschlagtem Gas und einem zweiten Auslaß (52) zur Aufnahme des abgetrennten anästhetischen Mittels aufweisen.

2. Gerät nach Anspruch 1, wobei der genannte Bereich ein Anästhetikumzufuhrmittel zum Zuführen von Anästhetikum zum Patienten umfasst, wodurch eine Rezirkulation des getrennten anästhetischen Mittels herbeigeführt wird.

3. Gerät nach Anspruch 1 oder 2, mit Entsorgungsmitteln, die wirkungsmäßig mit dem ersten Auslaß (48) der Trennmittel verbunden sind, um an anästhetischem Mittel erschöpftes druckbeaufschlagtes Gas zur Freisetzung in die Atmosphäre zu einer Stelle zu leiten, die entfernt von der den Patienten umgebenden Atmosphäre liegt.

4. Gerät nach einem der Ansprüche 1 bis 3, mit Druckfühlermitteln (28) zum Überwachen des Drucks innerhalb des Pufferbehälters (26) und zum Bewirken des Betriebs des Verdichters (34) nur bei Erfassung eines oberhalb Atmosphärendruck liegenden Drucks.

5. Gerät nach Anspruch 4, weiter mit Steuermitteln (30) zum Empfangen eines Signals von den Druckfühlermitteln (28) und zum Einleiten einer Steuerung des Betriebs des Verdichters nach Bedarf.

6. Gerät nach einem der Ansprüche 1 bis 5, wobei der Verdichter (34) zum Verdichten des ausgeatmeten Gases auf einen Druck betreibbar ist, der gleich oder größer einer Atmosphäre Überdruck ist.

7. Gerät nach einem der Ansprüche 1 bis 6, mit einem Gasanalysator (56), der wirkungsmäßig zum Überwachen der Reinheit des abgetrennten anästhetischen Mittels angeschlossen und wirkungsmäßig mit einem Anästhetikumzufuhrgeräts (10) so verbunden ist, daß das rezirkulierte anästhetische Mittel mit Zubereitungssauerstoff und frischem anästhetischem Mittel zur Zufuhr zum Patienten rückmischbar ist.

8. Gerät nach einem der Ansprüche 1 bis 7, wobei der Pufferbehälter (26) einen zusammendrückbaren/expandierbaren Pufferbeutel aufweist.

9. Gerät nach einem der Ansprüche 1 bis 8, wobei das anästhetische Mittel Distickstoffoxid umfasst.

10. Verfahren zur Rückgewinnung eines anästhetischen Mittels, das von einem Patienten ausgeatmet wird, wobei das Verfahren das Aufnehmen und zeitweilige Speichern der Ausatmung eines Patienten in einem Pufferbehälter (26), das Leiten des ausgeatmeten Gases aus dem Pufferbehälter (26) zu einem Trenngerät (40) zum Trennen des anästhetischen Mittels von dem ausgeatmetem Gas und das Leiten des abgetrennten anästhetischen Mittels in einen anderen Bereich als in die den Patienten umgebende Atmosphäre umfasst, **dadurch gekennzeichnet, daß** das ausgeatmete Gas aus dem Pufferbehälter (26) zu einem Verdichter (34) geleitet wird, der das ausgeatmete Gas verdichtet, und aus dem Verdichter zum Trenngerät (40) geleitet wird, das ein Membrantrenngerät ist.

## Revendications

1. Dispositif pour la récupération d'un agent anesthésique exhalé par un patient, le dispositif comprenant :
une cuve tampon (26) pour recevoir l'exhalaison d'un patient et stocker temporairement celle-ci ;
des moyens de séparation (40), pour recevoir le gaz exhalé et séparer l'agent anesthésique de celui-ci et pour diriger l'agent anesthésique séparé vers une zone autre que l'atmosphère entourant le patient, ***caractérisé en ce que*** le dispositif possède un compresseur (34) situé en aval de la cuve tampon (26) et relié opérationnellement à celle-ci afin de recevoir et comprimer le gaz exhalé et ***en ce que*** les moyens de séparation comprennent un dispositif de séparation par membrane relié opérationnellement au compresseur (34) pour recevoir le gaz exhalé comprimé sur son côté tubulure et configuré pour permettre à l'agent anesthésique de traverser la membrane (44) vers le côté calandre (46) de celle-ci, les moyens de séparation comprenant de plus une première sortie (48) pour recevoir du gaz sous pression appauvri en agent anesthésique et une deuxième sortie (52) pour recevoir l'agent anesthésique séparé.

2. Dispositif selon la revendication 1, dans lequel ladite zone comprend un moyen d'alimentation en anesthésique pour fournir un anesthésique au patient, pour ainsi assurer un recyclage dudit agent anesthésique séparé.

3. Dispositif selon la revendication 1 ou la revendication 2, comprenant des moyens d'évacuation reliés opérationnellement à la première sortie (48) des moyens de séparation pour diriger du gaz sous pression appauvri en agent anesthésique afin de le relâcher dans l'atmosphère en un point éloigné de l'atmosphère entourant le patient.

4. Dispositif selon l'une quelconque des revendications 1 à 3, comprenant des moyens de capteur de pression (28) pour réguler la pression dans la cuve tampon (26) et pour ne provoquer le fonctionnement du compresseur (34) qu'à la détection d'une pression supérieure à la pression atmosphérique.

5. Dispositif selon la revendication 4, comprenant de plus des moyens de commande (30) pour recevoir un signal venant desdits moyens de capteur de pression (28) et pour enclencher la commande du fonctionnement du compresseur quand et lorsque nécessaire.

6. Dispositif selon l'une quelconque des revendications 1 à 5, dans lequel le compresseur (34) est utilisable pour comprimer le gaz exhalé à une pression égale ou supérieure à une atmosphère au-dessus de l'atmosphérique.

7. Dispositif selon l'une quelconque des revendications 1 à 6 comprenant un analyseur (56) de gaz relié opérationnellement pour surveiller la pureté de l'agent anesthésique séparé et relié opérationnellement à un dispositif (10) d'alimentation en anesthésique afin de permettre à l'agent anesthésique recyclé d'être à nouveau mélangé avec de l'oxygène d'appoint et de l'agent anesthésique frais pour fourniture au patient.

8. Dispositif selon l'une quelconque des revendications 1 à 7, dans lequel la cuve tampon (26) comprend un tampon fait d'un sac souple dégonflable/expansible.

9. Dispositif selon l'une quelconque des revendications 1 à 8, dans lequel l'agent anesthésique comprend du protoxyde d'azote.

10. Procédé de récupération d'un agent anesthésique exhalé par un patient, le procédé comprenant la réception et le stockage temporaire de l'exhalaison d'un patient dans une cuve tampon (26) ; l'envoi du gaz exhalé de la cuve tampon (26) vers un dispositif de séparation (40) afin de séparer l'agent anesthésique du gaz exhalé et diriger l'agent anesthésique séparé vers une zone autre que l'atmosphère entourant le patient, ***caractérisé en ce que*** le gaz exhalé provenant de la cuve tampon (26) est dirigé vers un compresseur (34) comprimant le gaz exhalé, et du compresseur est dirigé vers l'appareil de séparation (40) comprenant un dispositif de séparation par membrane.
